# EUROPEAN PATENT SPECIFICATION

(11) **EP 0 871 438 B1**
(45) Date of publication and mention of the grant of the patent: **27.02.2008**
(21) Application number: 96920846.1
(22) Date of filing: 20.06.1996
(51) Int. Cl.: A61K 31/19, C12P 7/56

(54) **USE OF ALPHA-HYDROXY ACIDS IN THE MANUFACTURE OF A MEDICAMENT FOR THE TREATMENT OF INFLAMMATION**
VERWENDUNG VON ALPHA-HYDROXYSÄUREN ZUR HERSTELLUNG EINES ARZNEIMITTELS FÜR DIE BEHANDLUNG VON ENTZÜNDUNGEN
UTILISATION D'ACIDES ALPHA-HYDROXY DANS LA PREPARATION D'UN MEDICAMENT POUR LE TRAITEMENT DE L'INFLAMMATION

(30) Priority: 21.06.1995 FI 953090; 20.06.1996 FI 962602
(43) Date of publication of application: 21.10.1998
(73) Proprietor: Oy Extracta Ltd., 00810 Helsinki (FI)
(72) Inventor: WESTERMARCK, Hakon, deceased (FI); HIETALA, Pentti, FIN-00660 Helsinki (FI); JAARMA, Maire, S-126 50 Hägersten (SE); SORSA, Timo, FIN-00200 Helsinki (FI); VAARA, Martti, FIN-00400 Helsinki (FI)
(74) Representative: Vastavuo, Juhani Veli
(86) International application number: PCT/FI1996/000363
(87) International publication number: WO 1997/000676

(56) References cited:
- EP-A- 0 273 202
- STN INTERNATIONAL, File CA, CHEMICAL ABSTRACTS, Volume 90, No. 23, 4 June 1979 (Columbus, Ohio, US), HIETALA, PENTTI K. et al: "Identification of Antimicrobial Alpha-Hydroxyacids in Lactobacillus Plantarum-Fermented Animal Protein"; & NUTR. METAB. (1979), 23(3), 227-34.

## Description

The present invention relates to the use of an alpha-hydroxy acid as defined in the annexed claims. According to the invention, the use of alpha-hydroxy acids is based on their inhibitory and bactericidal efficacy on micro-organisms and proteinases, particularly the inhibition of matrix metalloproteinases. Matrix metalloproteinases are proteolytic enzymes which hydrolyze the proteins of the basement membrane of connective tissue and inflammation transmitter substances.

The state of the art is described in, e.g., publication Nutrition and Metabolism 23 (3), pp. 227-234 (1979), according to which two alpha-hydroxy acids, namely, alpha-hydroxy-isovaleric acid (d,1-2-hydroxy-3-methyl butyric acid) and alpha-hydroxy-isocaproic acid (d,1-2-hydroxy-4-methyl valeric acid) are isolated from a solution. The solution is obtained from protein-rich animal-based waste through *Lactobacillus plantarum* fermentation. The isolation of these acids was initiated by the discovery that such a fermentation solution exhibits growth-inhibiting efficacy on two micro-organisms, namely, *Escherichia coli* and *Trichophyton sp.* In the present invention it has been found that these compounds are inhibitory to a large spectrum of micro-organisms.

Lactic acid bacteria and lactic acid are widely used to modify and preserve organic material such as nutrients and animal feed. Lactic acid is also used in the treatment of skin diseases.

According to US Pat. No. 5,389,679, lactic acid can be used in the treatment of lesions of the mouth, especially aphthosis. The etiology of these lesions of the mucous membrane is uncertain. Reference is herein made to *Streptococcus sanguis* bacterium, which may be a partial cause to the onset of this disease among other factors. The antimicrobial efficacy of lactic acid may be exerted via acidity alone, provided that any significant antimicrobial effect in the healing of aphthosis exists at all. Matrix metalloproteinases can also be found in these ulcerations. On this basis it is plausible to assume the inhibition of these proteinases to have an effect in the treatment of oral cavity lesions irrespective of whether a microbial infection is an essential cause in the occurrence of the lesions or not.

US Pat. No. 4,772,592 discloses improved preparations for the local treatment of acne. The active compounds described therein are esters formed by lactic acid with C₁ - C₄ alcohols. The active compounds are assumed to comprise free lactic acid and alcohol that are formed by lipases of bacterial origin after the esters have resorbed through the epidermis of the skin.

US Pat. No. 4,380,549 describes a large number of organic acids, their salts and other derivatives suited for the treatment of "dry skin". Acids occurring in the nature and made through synthetic pathways can be used for said purpose in different formulations. The pH of such formulations has been in the range 2.7 - 5.7. In cited US Pat. No. 4,380,549, the possible antimicrobial efficacy or inhibition of proteolytic enzymes exhibited by said compounds in the treatment of the "dry skin" syndrome is not discussed nor indicated in any form at all.

US Pat. No. 4,363,815 describes the use of alpha-hydroxy acids and alpha-type keto acids in conditions characterized by disturbed keratinization. Alpha hydroxy acids can be used for local treatment in these disorders. The effective compounds include all hydroxy and keto analogues of amino acids irrespective of whether the corresponding amino acids occur in the proteins or not. According to the present invention, alpha-hydroxy acids can be used for improving the anti-inflammatory efficacy of corticosteroids and as a stabilizer of dithranol in formulations suited for local treatment. The antimicrobial efficacy of these acids and their inhibitory efficacy on matrix metalloproteinases have not been taken into account as a potential mechanism of action.

The present invention describes a novel use of alpha-hydroxy acids acting through the general antimicrobial efficacy of these compounds and via their inhibitory efficacy on matrix metalloproteinases. The characterizing specifications of the invention are disclosed in the annexed claims. Their efficacy on ingress of pathogenic microbes into tissues is divided into two steps: growth of organisms is inhibited by alpha-hydroxy acids, and inhibition of matrix metalloproteinases in the host cells prevents the escalation of the inflammation.

The invention offers significant benefits.

The alpha-hydroxy acids, or mixtures thereof, utilized in accordance with the invention can be either synthesized chemically or produced biotechnically into, e.g., a fermentation solution containing the bacterium. An essential requirement herein is that the acid contains at least 4 carbon atoms and/or a compound or compounds incorporating a branched carbon chain or an aromatic or other suitable substituent. Particularly advantageous in this aspect are 2-hydroxy-3-methyl butyric acid, 2-hydroxy-4-methyl valeric acid or a mixture thereof.

Matrix metalloproteinases (MMP) are proteolytic enzymes that are produced by human and mammal cells to provide for the lysis of the external connective tissue and basement membrane of the cell when required (cf. Woessner F. Jr., Ann. N.Y. Acad. Sci. 732: 11-21, 1994). MMP enzymes are capable of disintegrating almost all proteins of the connective tissue external to the cell and the cell basement membrane as well as, during the acute phase of a disease, certain serine proteinase inhibitors of serum (that is, inhibitors and serpines such as alpha-1-antitrypsin) and inflammation transmitter substances (e.g., the alpha factor of tumor necrosis) (cf Krane S.M., Ann. N.Y. Acad. Sci. 732: 11-21, 1994). While the number of members in the MMP enzyme family today includes 14 different members produced by different genes, obviously new members of the MMP enzyme family will be identified in the long run (cf. Matrisian L.M.. Ann. N.Y. Acad. Sci. 732: 42-50, 1994).

In many human and mammal diseases (e.g., rheumatic, gingival, cancer, blood vessel, gastrointestinal, ear, eye, skin diseases and painful recurring ulcerations of the oral cavity), the increased production and activation of MMP enzymes in different types of cells which are either infected or surrounding/adjacent to such human and mammal inflamed lesions exceed the concentrations and inhibitory efficacy of the inhibitor proteins (alpha-2-macroglobulin) and the tissue inhibitors (TIMP molecules) of MMP enzymes produced by the organism itself. This often causes a local and/or systemic tissue damage or lesions typical of the disease (cf. Sorsa et al., Ann. N.Y. Acad. Sci. 732: 112-131, 1994, Woessner J. Fr., Ann. N.Y. Acad. Sci. 732: 11-21, 1994, Häyrinen-Immonen et al., Int. J. Oral. Maxillfac. Surg. 22: 46-49, 1993, Häyrinen-Immonen et al., J. Oral. Pathol. Med. 23: 269-272, 1994), whereby the pathological changes are characterized by disintegration and damage of the connective tissue and basement membrane structure and adhesion as well as diseases and lesions of skin, gingival tissue, mucosa parts of mouth and the intestinal tract, cornea, cartilaginous and bone tissues. Simultaneously, the activities of serine proteinases (e.g., cathepsin-G) are elevated in the different diseases of humans and mammals (cf. Weiss, S.J., N. Engl. J. Med. 320: 365-376, 1989, Tervahartiala et al., J. Clin. Periodontol. 23: 68-75, 1996). Microbes capable of producing proteinases in conjunction with parodontites include:
- *Bacteroides forsythus*
- *Porphyromonas gingivalis*
- *Prevotella intermedia*
- *Fusobacterium nucleatum*
- *Treponema denticola*
- *Peptostreptococcus micros*
- *Actinobacillus actinomycetemcomitans*
- *Campylobacter rectus.*

The function of alpha-hydroxy acids according to the invention is to lower or prevent the occurrence of pathologically elevated activities and concentrations of matrix metalloproteinases and serine proteinases (cathepsin G) so that normal cellular activities and concentrations of these proteinases are maintained in the tissues of human and mammal organisms during diseases which usually involve elevated activities and concentrations of said enzymes. Alpha-hydroxy acids are suited for both treatment of inflammations in humans and mammals as well as eradication of multiresistant microbes from their carriers. The invention concerns the use of alpha-hydroxy acids in all pharmaceutically acceptable forms including tablets, capsules, powders, granules, syrups, elixirs and rectal suppositories. The tablets may be coated or uncoated. Besides the antimicrobially active component, the oral preparations and rectal suppositories may incorporate other pharmaceutically acceptable constituents. The invention further concerns locally applied preparations, ointments, solutions, dispersions, tinctures, drops and rectal suppositories that are used for both curative or preventive treatment of infections affecting skin, mucous tissues, ophthalmological, gynecological, otological, oral and other sites of infections as well as eradication of pathogenic and multiresistant microbes from their carriers.

Examples on the use of alpha-hydroxy acids for prevention of microbial infections in epithelic tissues were carried out using two acids: d,1-2-hydroxy-3-methyl butyric acid and d,1-2-hydroxy-4-methyl valeric acid. These acids are physiologically acceptable, since their complete catabolism in organic tissues has been verified by means of thorough investigations into their enzymatic reactions. According to current knowledge, their use must be limited to the skin, the oral cavity and the alimentary tract only. At the administration level used according to the invention, no systemic side-effects are to be expected. Inasmuch as is known from these acids, their resorption from the intestine takes place passively.

Therefore, their retention time in the intestine becomes long, which enhances the antimicrobial efficacy exerted by the acids in the gastrointestinal tract.

As the d,1-2-hydroxy-3-methyl butyric acid and the d,1-2-hydroxy-4-methyl valeric acid are soluble in both water and lipid-dissolving fluids, they will be distributed between the aqueous and lipid phases. Both of them are dissociable organic acids with pKₐ 3.80. Hence, the antimicrobially active form of these acids is the free acid, not the anion. Further, their antimicrobial efficacy is dependent on the pH. At pH 3-8, these acids are half-neutralized, whereby the buffering capacity of their solutions is at a maximum.

In the following, the invention will be described in greater detail with the help of the following examples and with reference to annexed drawings in which
Figure 1 is a plot of the growth inhibition of *E*. *coli* and *S. aureus* by HMV (d,1-2-hydroxy-4-methyl valeric acid);
Figure 2 is a plot of the inhibition of GCF (gingival crevicular fluid) collagenase activity by HMV (d,1-2-hydroxy-4-methyl valeric acid) and HMB (d,1-2-hydroxy-3-methyl butyric acid) with and without APMA (aminophenyl mercuric acetate).
Figure 3 is a plot of the dose-dependent inhibition of the catalytic activity of human gingival crevicular liquid collagenase (MMP-8 Inhibition) by means of alpha-hydroxy acids;
Figure 4 is a plot of the reduction/inhibition of the catalytic activity of human fibroblast gelatinase A (72 kD type IV collagenase MMP-2) and of human neutrophil gelatinase B (92 kD type IV collagenase MMP-9) by alpha-hydroxy-isocaproic acid;
Figure 5 is a plot of the reduction/inhibition of the catalytic activity of human fibroblast gelatinase A (MMP-2) by alpha-hydroxy-isovaleric acid;
Figure 6 is a plot of the inhibition of the catalytic activity of cathepsin-G isolated from human neatrophils; and
Figure 7 is a plot of the inhibition of the catalytic activity of cathepsin-G isolated from human neutrophils.

The following examples describe the effects of these alpha-hydroxy acids and their use in the treatment of inflammation. As the examples are given only to elucidate the invention, the applications of the invention are not limited thereto, but rather, the materials and uses described in the examples can be varied for different purposes within the scope and spirit of the invention disclosed in the annexed claims.

Abbreviations used in the examples:
HMV = d,1-2-hydroxy-4-methyl valeric acid
HMB = d,1-2-hydroxy-3-methyl butyric acid
MMP = matrix metalloprotemase, different types identified with a code number
GCF = gingival crevicular fluid
APMA = aminophenyl mercuric acetate.

### Example 1 (illustrative example, not part of the invention)

The antimicrobial efficacy of d,1-2-hydroxy-3-methyl butyric acid and d,1-2-hydroxy-4-methyl valeric acid was tested *in vitro.* The concentration of these compounds were adjusted to 4 mg/ml of typical culture medium solution of for each microbe listed below. The pH of the culture medium solutions was adjusted at pH 5.2. Growth inhibition was detected for the following microbes:
*Trichophyton sp.*
*Escherichia coli*
*Staphylococcus aureus*
*Salmonella typhimurium*
*Salmonella typhi 643 W*
*Streptococcus pyogenes*
*Streptococcus mutans*
*Klebsiella pneumoniae*
*Helicobacter pylori*
*Staphylococcus capitis MCS A 1 ATCC 27 482*
*Staphylococcus warneri RM 130 ATCC 27837*
*Staphylococcus lactis 15306*
*Staphylococcus muscae ARCC 121162*
*Staphylococcus xylosus KL 162*
*Staphylococcus simulans MK 148 ATCC 27848*
*Staphylococcus epidermis AW 269*
*Staphylococcus haemolyticus SM 131*
*Staphylococcus cohnii GH 137*
*Staphylococcus citreus 413 Manchester*
*Staphylococcus hominis KL 243 ATCC 27846*
*Staphylococcus saprophyticus TW 11*
*Staphylococcus epidermis 128 Lausanne*

### Example 2 (illustrative example, not part of the invention)

A mixture of d,1-2-hydroxy-4-methyl valeric acid and its sodium salt was prepared in mole ratio 1: 1. The mixture was homogenized and sifted through a 200-mesh sieve. The powder thus obtained was rubbed on irritated skin areas of patients infected by *Trichophyton,* twice a day, in the morning and in the evening. The *Trichophyton* infection was healed in two weeks.

### Example 3 (illustrative example, not part of the invention)

Growth medium: the bacteria were grown on trypticase-soya-meat extract medium (BBL) having its pH adjusted acid (at pH 5.5). Into this medium were added increasing amounts of d,1-2-hydroxy-4-methyl valeric acid (HMV) and adjusted again at pH 5.5.

Bacterial strains: the bacteria used in the example were strains *Staphylococcus aureus ATCC 25923* and *Escherichia coli ATCC 23682* obtained from American Type Culture Collection.

Measurement of antibacterial activity: bacterial colonies grown on blood agar dishes at 37 °C were suspended in sterile salt solution (0.9 % NaCl) and a further dilution of this suspension (into sterile salt solution) was used as a bacterial graft for inoculating growth medium aliquots of 200 µl pipetted into wells of microtiter plates (the size of the graft was about 10⁵ colony-forming units (CFU) per milliliter). The plates were next incubated at 37 °C for 24 h (*S. aureus)* or 48 h *(E. coli).* Subsequently, the bacterial growth was determined by measuring the absorbance of each well at A 405 nm wavelength using Titertek Multiscan spectrometer (vendor Labsystems Oy, Helsinki, Finland). One well of the microtiter plate containing noninoculated culture medium only was used as the zero reference of the spectrometer.

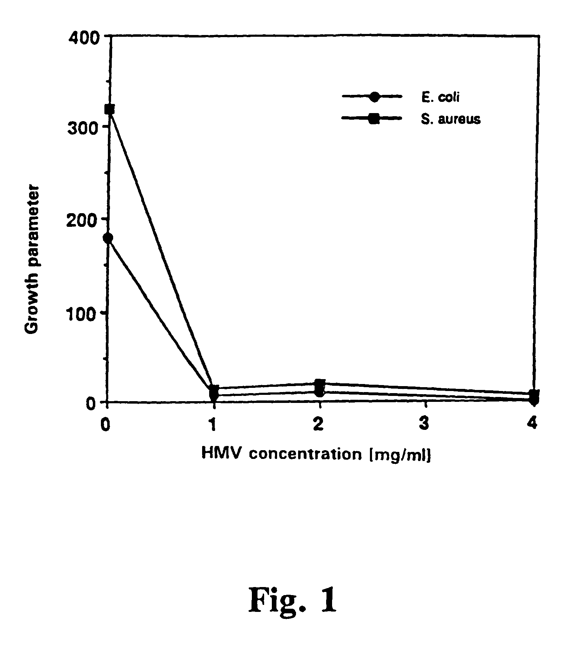

Results: Fig. 1 is a plot of the test results. The growth of *Staphylococcus aureus* and *Escherichia coli* is plotted in relative units in trypticase-soya-meat extract culture medium (at pH 5.5) containing 1-2-hydroxy-4-methyl valeric acid. The inhibitory effect of d,1-2-hydroxy-4-methyl valeric acid on the growth of both *S. aureus* and *E. coli* is seen at an HMV level of 1 mg/ml culture medium.

### Example 4 (illustrative example, not part of the invention)

Preparations and buffer solutions: preparation A (0.625 % HMV) contained one part of 2.5-percent d,1-2-hydroxy-4-methyl valeric acid (HMV) ointment (pH 3.9) and three parts of sterile distilled water. Preparation B (control preparation) contained one part of acid ointment (pH 3.8) and three parts of distilled water. The phosphate-buffered sterile salt solution (pH 7.2, PBS) contained 0.9 % NaCl and 0.01 M potassium phosphate buffer solution.

Bacterial strains: the bacterial strains in the test were *Streptococcus pyogenes ATCC 19615, Staphylocaccus aureus ATCC 25923, Pseudomonas aeruginosa ATCC 27853* and *Escherichia coli ATCC 23682* obtained from American Type Culture Collection. *Stenotrophomonas (Xanthomonas) maltophilia KY 3 728, Acinetobacter sp. KY 109* and *Propionibacterium acnes KY 16409* were clinical isolations obtained from the strain collection of Helsinki University, Unit of Diagnostic Bacteriology (Helsinki, Finland).

Assay of antibacterial activity: bacterial colonies, which were grown on blood agar dishes at 37°C under anaerobic conditions *(P. acnes),* in a candle jar *(Str. pyogenes)* or aerobically (other strains), were suspended in sterile salt solution (0.9 % NaCl) and a further dilution of this suspension (into sterile salt solution) was used as a bacterial graft for inoculating 1 ml aliquots of preparations A and B (the size of the graft was about 10⁵ colony-forming units (CFU) per milliliter). The inoculated aliquots of the preparations were next incubated at 37 °C for 18 h. Subsequently, the amounts of surviving bacteria were determined by first transferring a 5µl sample into 500 µl of PBS solution, then diluting this solution into PBS in increasing powers n of ten per ml ofPBS and finally inoculating blood agar dishes with these successive series of dilutions. The colonies were allowed to grow at 37 °C for 3 days *(P. acnes)* or 24 h (other strains) under the growth conditions described above.

Results: Table 1 below shows the test results. Preparation A (0.625 % HMV) was able to suppress the viability of the following bacterial strains by a factor of ≥ 100: *Streptococcus pyogenes ATCC 19615, Staphylococcus aureus ATCC* 25923, *Pseudomonas aeruginosa ATCC 27853, Escherichia coli ATCC 23682, Stenotrophomonas maltophilia KY 3728* and *Acinetobacter sp. KY 109.* The viability of *Propionibacterium acnes KY 16409* strain was suppressed by a factor of ≥ 10.

**Table 1. Amount of viable bacterial cells (expressed as colony-forming units per milliliter, CFU/ml), after the bacteria were treated by at 37 °C for 18 h using preparation A (2.5 % HMV ointment, pH 3.9, diluted with water in ratio 1:3 v/v) and preparation B (control ointment, pH 3.8, diluted with water in ratio 1:3 v/v). The amount of bacterial graft was 10⁵ CFU/ml.**

| Bacterium | Preparation A (HMV) | Preparation B (control) |
|---|---|---|
| *Streptococcus pyogenes ATCC 19615* | < 10³ | < 10³ |
| *Staphylococcus aureus ATCC 25923* | < 10³ | 800 x 10³ |
| *Pseudomonas aeruginosa ATCC 27853* | < 10³ | 2500 x 10³ |
| *Escherichia coli ATCC 23682* | < 10³ | 3000 x 10³ |
| *Stenotrophomonas (Xanthomonas) maltophilia 3728* | < 10³ | 2 x 10³ |
| *Acinetobacter sp. 109* | < 10³ | 2000 x 10³ |
| *Propionibacterium acnes KY 16409* | 4 x 10³ | < 10³ |

### Example 5

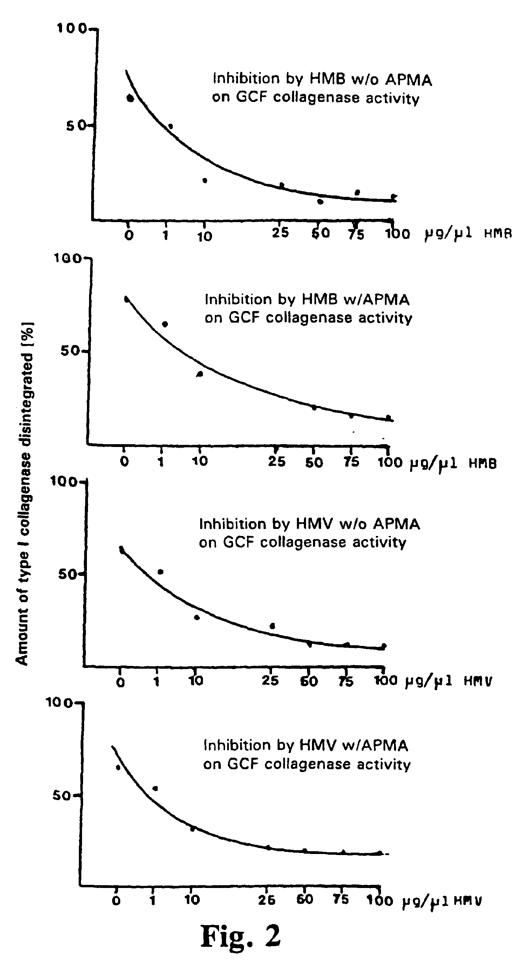

This example was carried out to test the dose-dependent inhibition of the catalytic activity of human neutrophil collagenase (MMP-8 inhibition) by means of alpha-hydroxy acids. In the test, human neutrophil collagenase (MMP-8, 500 ng, both without and with activation with 1 mM aminophenyl mercuric acetate; cf. Sorsa et al. Ann. N.Y. Acad. Sci. 732: 112-131, 1994) was pretreated (at 37 °C for 1 h) with a buffer solution (using TNC buffer solution containing 50 mM Tris-HCl, 0.15 M NaCl. 1 mM CaCl₂, pH 7.8) and with 1-100 µg/µl alpha-hydroxy-isocaproic acid (d,1-2-hydroxy-4-methyl valeric acid, titrated at pH 7.8) and with alpha-hydroxy isovaleric acid (d,1-2-hydroxy-3-methyl butyric acid, titrated at pH 7.8) and subsequently incubated in TNC buffer solution with 1.5 µM soluble native type I collagen at ambient temperature (22 °C) for 12 h. Next, the samples were boiled at 100 °C for 5 min in Laemmli buffer solution and analyzed using 8-10 % sodium-dodecyl-sulfate-polyacrylamide gel electrophoresis (cf. Sorsa et al., Ann. N.Y. Acad. Sci. 732: 112-131, 1994). The SDS-PAGE gels were stained with 1.2 mM Coomassie Brilliant Blue and the excess stain was washed away with a mixture containing 10 % acetic acid and 5 % methanol, after which the gels were measured using a laser densitometer. The catalysis of type I collagen induced by human neutrophil collagenase MMP-8 was measured as percent (%) of type I collagen disintegrated. Both the alpha-hydroxy-isocaproic acid and the alpha-hydroxy-isovaleric acid were found to exert significant inhibition of human MMP-8 already at a concentration level of 10µg/µl. The test result is shown in Fig. 2.

### Example 6

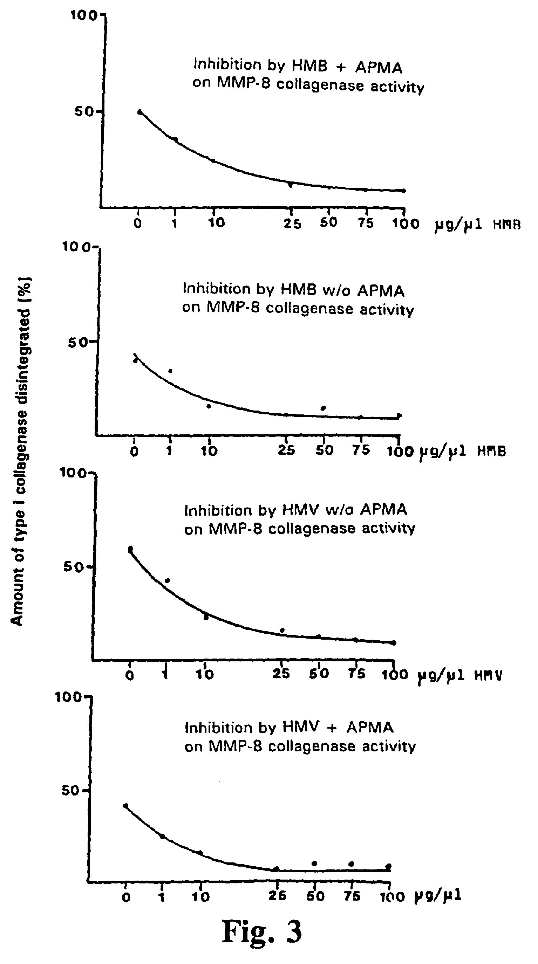

This example was carried out to test the dose-dependent inhibition of the catalytic activity of human gingival crevicular liquid collagenase (MMP-8 inhibition) by means of alpha-hydroxy acids (for *in vivo* form of MMP-8, cf. Sorsa et al., Ann. N.Y. Acad. Sci. 732: 112-131, 1994, Tervahartiala et al., J. Clin. Periodontol. 23: 68-75, 1996). In the test, human gingival crevicular liquid collagenase (MMP-8, 5 µg, both without and with activation with 1 mM aminophenyl mercuric acetate; cf. Sorsa et al. Ann. N.Y. Acad. Sci. 732: 112-131, 1994) was pretreated (at 37 °C for 1 h) with a buffer solution (using TNC buffer solution containing 50 mM Tris-HCl, 0.15 M NaCl, 1 mM CaCl₂, pH 7.8) and with 1-100 µg/µl alpha-hydroxy-isocaproic acid (d,1-2-hydroxy-4-methyl valeric acid, titrated at pH 7.8) and with alpha-hydroxy isovaleric acid (d,1-2-hydroxy-3-methyl butyric acid, titrated at pH 7.8) and subsequently incubated in TNC buffer solution with 1.5 µM soluble native type I collagen at ambient temperature (22 °C) for 12 h. Next, the samples were boiled at 100 °C for 5 min in Laemmli buffer solution and analyzed using 8-10 % sodium-dodecyl-sulfate-polyacrylamide gel electrophoresis (cf. Sorsa et al., Ann. N.Y. Acad. Sci. 732: 112-131, 1994). The SDS-PAGE gels were stained with 1.2 mM Coomassie Brilliant Blue and the stain was washed away with a mixture containing 10 % acetic acid and 5 % methanol, after which the gels were measured using a laser densitometer. The catalysis of type I collagen induced by human gingival crevicular collagenase MMP-8 was measured as percent (%) of type I collagen disintegrated. Both the alpha-hydroxy-isocaproic acid and the alpha-hydroxy-isovaleric acid were found to exert significant inhibition of human MMP-8 already at a concentration level of 10µg/µl. The test result is shown in Fig. 3.

### Example 7

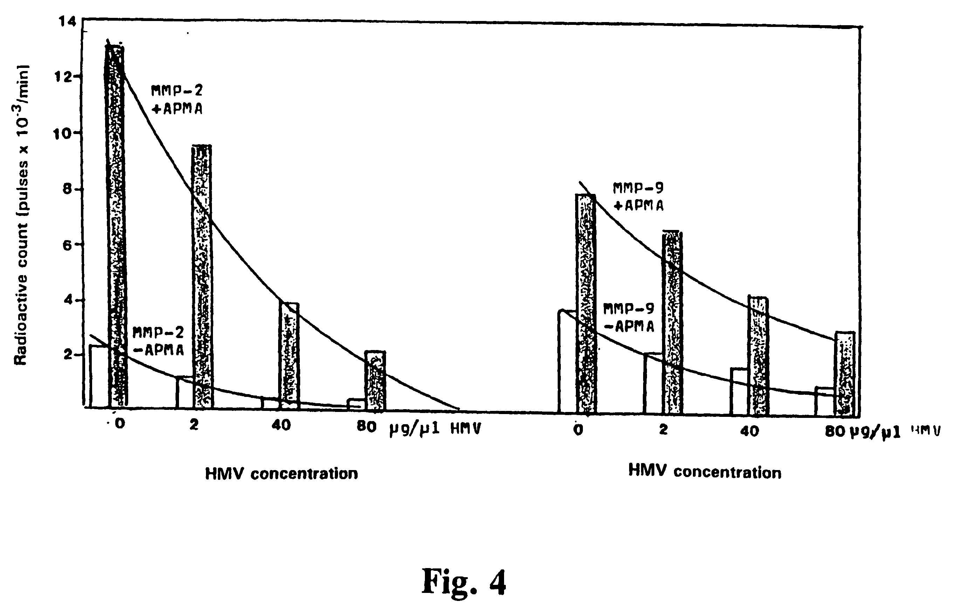

This example was carried out to test the reduction/inhibition of the catalytic activity of human fibroblast gelatinase A (72 kD type IV collagenase MMP-2) and of human neutrophil gelatinase B (92 kD type IV collagenase MMP-9) by alpha-hydroxy-isocaproic acid (d,1-2-hydroxy-4-methyl valeric acid, titrated at pH 7.8). In the test, 100 ng purified MMP-2 and 65 ng purified MMP-9 (both without and with activation with I mM aminophenyl mercuric acetate) were pretreated (at 37 °C for 1 h) with a buffer solution (using TNC buffer solution containing 50 mM Tris-HCl. 0.15 M NaCl, 1 mM CaCl₂, pH 7.8) and with 2-80 µg/µl alpha-hydroxy-isocaproic acid (d,1-2-hydroxy-4-methyl valeric acid, titrated at pH 7.8) and subsequently incubated with 1.5 µM ¹²⁵I-labelled radioactive type I gelatin (at 37 °C for 1 h), after which the nondisintegrated gelatin substrate was precipitated with 20 % trichloro acetic acid, and the radioactivity of the supernatant phase was measured to obtain an assay of the disintegrated gelatine substrate (cf. Westerlund et al., J. Dent. Res., in press, 1996). The alpha-hydroxy-isocaproic acid was found to exert significant inhibition of human gelatinase A (MMP-2) and gelatinase B (MMP-9) at a concentration level of 10µg/µl. The test result is shown in Fig. 4.

### Example 8

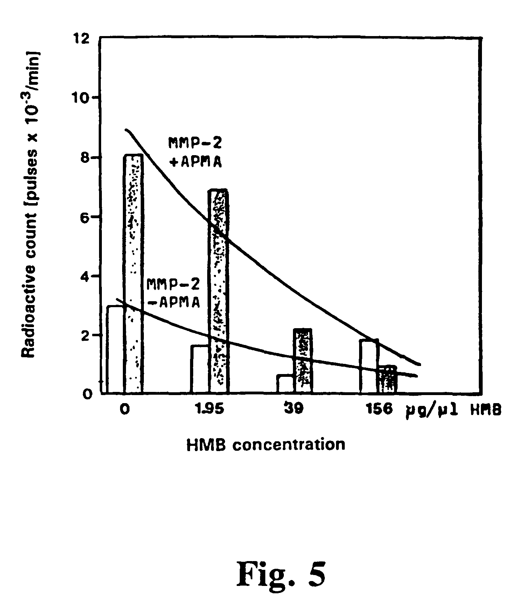

This example was carried out to test the reduction/inhibition of the catalytic activity of human fibroblast gelatinase A (72 kD type IV collagenase MMP-2) by alpha-hydroxy-isovaleric acid (d,1-2-hydroxy-3-methyl butyric acid, titrated at pH 7.8). In the test, 100 ng purified MMP-2 (both without and with activation with 1 mM aminophenyl mercuric acetate) was pretreated (at 37 °C for 1 h) with a buffer solution (using TNC buffer solution containing 50 mM Tris-HCl, 0.15 M NaCl, 1 mM CaCl₂, pH 7.8) and with 2-80 µg/µl alpha-hydroxy-isovaleric acid (d,1-2-hydroxy-3-methyl butyric acid, titrated at pH 7.8) and subsequently incubated with 1.5 µM ^{12S}I-labelled radioactive type I gelatin (at 37 °C for 1 h), after which the nondisintegrated gelatin substrate was precipitated with 20 % trichloroacetic acid, and the radioactivity of the supernatant phase was measured to obtain an assay of the disintegrated gelatine substrate (cf. Westerlund et al., J. Dent. Res., in press, 1996). The alpha-hydroxy-isocaproic acid was found to exert significant inhibition of human gelatinase A (MMP-2) at a concentration level of 10µg/µl. The test result is shown in Fig. 5.

### Example 9

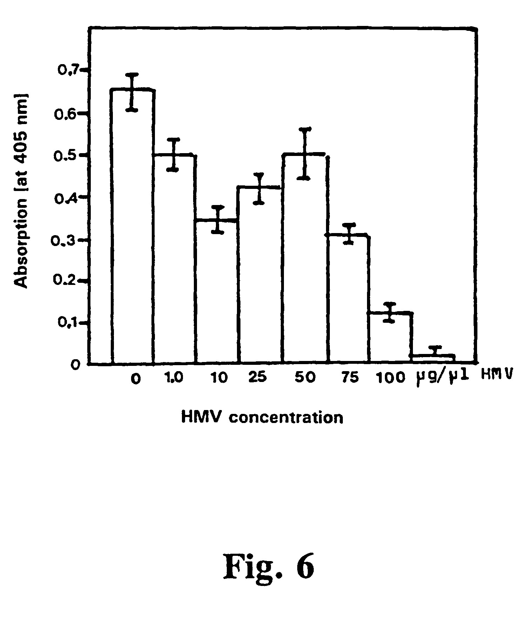

This example was carried out to test the inhibition of the catalytic activity of cathepsin-G isolated from human neutrophils. In the test, cathepsin-G (10 ng) was pretreated (at 37 °C for 1 h) with TNC buffer solution and with 1.0-100 µg/µl alpha-hydroxy-isocaproic acid (d,1-2-hydroxy-4-methyl valeric acid, titrated at pH 7.8) and subsequently the enzyme solutions were incubated with 1 mM succinyl-alanyl-alanyl-phenylalanyl-paranitroanilide (SAAPNA) peptide substrate at 37 °C for 2 h. The cathepsin-G activities were measured as increase of absorbance at 405 nm wavelength and plotted as either relative changes in absorbance and/or international units of enzyme activity (cf. Tervahartiala et al., J. Clin. Periodontol. 23: 68-75, 1996). Alpha-hydroxy-isocaproic acid was found to exert effective inhibition of the activity of cathepsin-G at a concentration level 10-50 µg/µl. The test result is shown in Fig. 6.

### Example 10

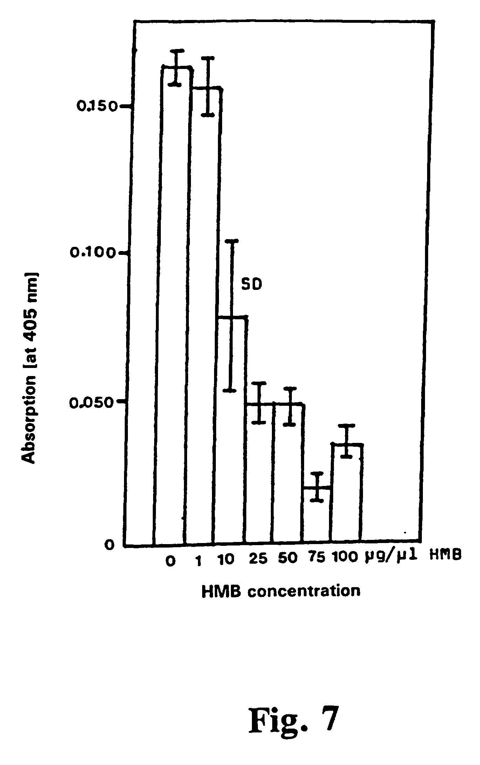

This example was carried out to test the inhibition of the catalytic activity of cathepsin-G isolated from human neutrophils. In the test, cathepsin-G (10 ng) was pretreated (at 37 °C for 1 h) with TNC buffer solution and with 1.0-100 µg/µl alpha-hydroxy valeric acid (d,1-2-hydroxy-3-methyl butyric acid, titrated at pH 7.8) and subsequently the enzyme solutions were incubated with 1 mM succinyl-alanyl-alanyl-phenylalanyl-paranitroanilide (SAAPNA) peptide substrate at 37 °C for 2 h. The cathepsin-G activities were measured as increase of absorbance at 405 nm wavelength and plotted as either relative changes in absorbance and/or international units of enzyme activity (cf. Tervahartiala et al., J. Clin. Periodontol. 23: 68-75, 1996). Alpha-hydroxy-isovaleric acid was found to exert effective inhibition of the activity of cathepsin-G at a concentration level 10-50 µg/µl. The test result is shown in Fig. 7.

### Example 11

Study A. This part of the example was carried out to test the effect of irrigation/flushing treatment with 2 % d,1-2-hydroxy-3-methyl butyric acid (HMB) and subsequent rinsing on the health condition of gums and mouth of parodontitis patients. The parodontitis patients participating the study were selected according to the following clinical criteria: deep gingival crevices (depth >4 mm), amount of gingival haemorrhages, visible plaque index, retentive tartar and bone resorption detected from x-ray pictures to be related to the deep gingival crevices in parodontitis conditions. 40 parodontitis patients were given flushing treatment with 2 % HMB, while a reference/control group (30 patients) received irrigation/flushing treatment with Sol. Rotocani. Sol. Polymineroli, and Sol. Maraslavini. After conventional mechanical removal of tartar, the test patients were given irrigation/flushing treatment with 2 % HMB, and subsequently, each patient was given an aliquot of 2 % HMB preparation to be used for mouth rinsing twice a day for 2 weeks; correspondingly, the reference/control group (30 patients) was treated with conventional mechanical removal of tartar and the initial irrigation/flushing treatment of gingival crevices was performed using Sol. Maraslavini. Sol. Rotokani and Sol Polymineroli preparations, after which the patients were instructed to perform mouth rinsing with water only. Clinical and microbiological studies were performed before and after the treatment. The microbiological growth tests were carried out on blood agar, chocolate agar and Tsistevits salt agar dishes. When it was necessary to isolate a pure culture on a differentiating dish, a new culture was grown on a conventional agar dish, and in unclear cases, the new cultures were grown on differentiating dishes complemented with additional biochemical and fermentative tests as required. The results show that the HMB preparation has a two-way action: firstly, the HMB preparation eradicated the pathogenic microbial flora present in the gingival crevices of the parodontitis patients entirely in about 40 % of the cases, and in some cases, the suppression of the microbial flora was better by orders of magnitude as compared with the reference/control group. Secondly, the HMB preparation altered the spectrum of the pre-treatment pathogenic microbial flora of the gingival crevices of the parodontitis patients toward apathogenic direction.

The purpose of this test was to assess the effect and function of the HMB preparation on the health condition of the gums and the mucosa of the oral cavity over a follow-up period of 2 to 3 months subsequent to a systematic flushing treatment carried out for 2 weeks. It was found that the HMB preparation has an antimicrobial effect lasting 3 months after the treatment. The clinical inflammation indices (gingival haemorrhages and visible plaque indices) characterizing the health condition of the gingival tissue are lowered significantly by virtue of the HMB treatment/prophylaxy. As these symptoms associated with the progress of parodontitis promote the further development of the disease, the HMB preparation may be capable of preventing the progress of gingivitis (and other gingival diseases) and parodontitis. When examined immediately after the completion of the treatment series, 40 % of the patients were found free from pathogenic microflora, and after 3 months, this figure was still 27.5 %. As compared with the control group, the result was 1.5-fold better.

The HMB preparation was capable of shifting the spectrum of the pathogenic microflora toward apathogenic direction. A particularly strong suppressing effect of the HMB preparation was found on streptococcal strains *(Streptococcus viridans and mutans).* For A-haemolytic streptococci, the HMB preparation was less effective: their number was reduced quantitatively, but viable cocci were still detected. The HMB preparation had also a strong antibacterial efficacy on *E. coli.*

Subjectively, parodontitis patients treated with HMB irrigation experienced a clean and light feeling in their mouths. In conclusion, the tests proved that a 2 % solution of the HMB preparation is suited for active treatment in the irrigation cleaning of deep gingival crevices (depth > 4mm) due to parodontitis, followed by extended use of the 2 % solution of the HMB preparation as a mouthwash, particularly as a general prophylaxy against recurrent parodontitis, and in the treatment of and as prophylaxy against recurrence of parodontitis for patients suffering from severe systemic diseases (diabetes, leukemia, arthrites, cancers, vascular diseases and chronic illnesses), as well as general prophylaxy in order to establish a inflammation-resistant condition in the mouth.

### Test results:

1. Control group, microbiological status of patients prior to treatment:

| | |
|---|---|
| 30 % having | α-haemolytic streptococci |
| 20 % having | apathogenic saprophytes |
| 20 % having | Escherichia coli |
| 20 % having | different types of bacteroid microbes |
| 10 % having | haemolytic haemophils (Haemophilus influenzae). |

Control group, microbiological status of patients after treatment:

| | |
|---|---|
| 18 % having | no growth of micro-organisms |
| 27 % having | α-haemolytic streptococci |
| 18 % having | growth of *Streptococcus viridans* |
| 10 % having | β-haemolytic streptococci |
| | *(Streptococcus pyogenes)* |
| 10 % having | apathogenic saprophytes. |
| 10 % having | apathogenic mixed flora. |

2. Test group, microbiological status of patients prior to treatment with HMB::

| | |
|---|---|
| 46 % having | Fam. *Streptococeae* |
| 15 % having | *Escherichia coli* |
| 12 % having | apathogenic mixed flora |
| 10 % having | bacteroid microbes |
| 5 % having | *Haemophilia haemolyticus* |
| *5%* having | no bacteriological flora |
| 8% having | micrococci. |

Test group, microbiological status of patients after treatment with a 2 % solution of the HMB preparation:

| | |
|---|---|
| 39 % having | no bacteriological flora |
| 18 % having | *Streptococcus viridans,* only a few cells thereof |
| 18 % having | apathogenic mixed flora |
| 8% having | α-haemolytic streptococci |
| 8% having | saprophytes, only a few cells |
| 5 % having | β-haemolytic streptococci, only a few cells |
| 3 % having | micrococci, only a few cells |
| 3 % having | treatment uncompleted. |

Study B. This part of the example was carried out to test the efficacy of a 2 % solution of the HMB preparation on the composition of the microbial flora in the toothbrushes of parodontitis patients prior and after the treatment.

A microbiological study was carried out on samples taken from the gaps between the bristles of patient toothbrushes. Microbes belonging to the group of streptococci and the *E. coli* group were founds abundantly prior to the treatment. Treatment with the 2 % solution of the HMB preparation lowered the count of these microbes significantly. As known the interbristle gaps in toothbrushes act as an undisputable retention site for parodontopathogenic and other oral cavity microbes. Furthermore, as toothbrushes are conventionally not washed and dried sterile after use thus forming a moist environment of highly favourable growth, carrier and culture medium substrate for oral cavity bacteria, the advantageous results obtained herein complement favourably the recommendable procedures of routine washing and disinfection of toothbrushes.

## Claims

1. Use of an alpha-hydroxy acid containing at least 4 carbons and having a branched carbon chain, or of a mixture of said acids, an antimicrobial and proteinase-inhibiting component in the manufacture of a pharmaceutical product for treatment of inflammation in animals and humans.

2. Use of an alpha-hydroxy acid as defined in claim 1 in product having antimicrobial and proteinase-inhibiting efficacy wherein said alpha-hydroxy acid is 2-hydroxy-3-methyl butyric acid, 2-hydroxy-4-methyl valeric acid, or a mixture thereof, in the manufacture of a pharmaceutical product for treatment of inflammation in animals and humans.

## Patentansprüche

1. Verwendung einer Alpha-Hydroxysäure enthaltend mindestens 4 Kohlenstoffatome und mit einer verzweigten Kohlenstoffkette, oder einer Mischung von diesen Säuren als antimikrobieller und Proteinase inhibierender Bestandteil für die Herstellung eines pharmazeutischen Produktes zur Behandlung von Entzündungen bei Tieren und Menschen.

2. Verwendung einer Alpha-Hydroxysäure nach Anspruch 1 in einem Prdoukt mit antimikrobieller und Proteinase inhibierender Wirksamkeit, wobei die Alpha-Hydroxysäure 2-Hydroxy-3-Methylbutylsäure, 2-Hydroxy-4-Methylvaleriansäure oder eine Mischung davon ist, für die Herstellung eines pharmazeutischen Produktes zur Behandlung von Entzündungen bei Tieren und Menschen.

## Revendications

1. Utilisation d'un acide alpha-hydroxy contenant au moins 4 atomes de carbone et ayant une chaîne carbonée ramifiée, ou d'un mélange desdits acides, d'un composant antimicrobien et inhibant la protéinase dans la préparation d'un médicament pour le traitement d'une inflammation chez des animaux et des êtres humains.

2. Utilisation d'un acide alpha-hydroxy selon la revendication 1 dans un produit ayant une efficacité antimicrobienne et inhibant la protéinase, dans laquelle ledit acide alpha-hydroxy est l'acide 2-hydroxy-3-méthylbutyrique, l'acide 2-hydroxy-4-méthyl-valérique ou un mélange de ceux-ci, dans la préparation d'un médicament pour le traitement d'une inflammation chez des animaux et des êtres humains.
